# EUROPEAN PATENT APPLICATION

(11) **EP 3 097 966 A1**
(43) Date of publication of application: **30.11.2016**
(21) Application number: 14879552.9
(22) Date of filing: 23.01.2014
(51) Int. Cl.: B01D 39/16, B01D 46/10, D04G 1/00, A61L 9/00, B01J 23/40, B01J 35/06, D03D 15/00

(54) **FUNCTIONAL AIR FILTER**

(71) Applicant: Taiyo Co., Ltd, Chiyoda-ku, Tokyo 101-0021 (JP)
(72) Inventor: MUKAI, Kiyotoshi, Tokyo 101-0021 (JP); HAYASHI, Yasuhiro, Tokyo 101-0021 (JP); SANO, Masataka, Hamamatsu-shi Shizuoka 431-1102 (JP)
(74) Representative: Vossius & Partner Patentanwälte Rechtsanwälte mbB
(86) International application number: PCT/JP2014/051418
(87) International publication number: WO 2015/111172

(57) **Abstract**

Provided is a functional air filter which can maintain a sufficient function of suppressing breeding of mold and undesired bacteria for a long period thus being hygienic, exhibiting high safety and possessing deodorizing property. In a functional air filter which is manufactured by, at intersections between wefts and warps made of a thermoplastic sheath-core type composite monofilament which is a composite fiber consisting of a core material and a sheath material made of a resin having a lower melting point than the core material, heat-fusing the sheath materials to each other, the composite monofilament is configured such that some particles blended into the sheath material are exposed from a surface of the sheath material. The particle is a mixed particle where fine particles are fixedly adhered to a surface of a coarse particle.

## Description

### Technical Field

The present invention relates to an air filter which is used in a state where the air filter is mounted on a ventilation port or the like of an air conditioner, an air cleaner or the like, and more particularly to a functional air filter which can maintain a hygienic function of suppressing breeding of mold, undesired bacteria and the like for a long period, exhibits high safety and has deodorizing property.

### Background Art

Along with the rise of tendency of placing importance on health in recent years, products referred to as antibacterial commodities have been popularly available on markets.

Such tendency is observed not only with respect to products which consumers can directly touch with their hands. For example, treatment which suppresses the breeding of mold and undesired bacteria is applied also to an air filter incorporated into an air conditioner or an air cleaner which are requisite household commodities currently. Further, an attempt has been made to effectively clean air by imparting deodorizing function to a product in a method where the product is in contact with air.

The air filter of this type is, in general, a net fabric formed of monofilaments made of thermoplastic resin, and is formed by kneading a suitable amount of an additive made of a compound having antibacterial property such as an organic halogenated compound, an unsaturated carbonyl compound, an amide-based compound or a triazole-based compound, for example, into a material resin in a monofilament spinning stage.

However, these compounds exhibit poor heat resistance in general and hence, there is a case where these compounds are degenerated by decomposition depending on a heating temperature at the time of melt-spinning and hence, a particular care such as maximally lowering a molding temperature by using a particular raw material resin is required in many cases.

Further, in the air filter manufactured by such spinning, basically, particles exposed on surfaces of filaments exhibit antibacterial property by being dissolve and hence, the air filter has a drawback with respect to a point whether or not antibacterial property can be stably maintained with time. Further, among these compounds, there are some compounds which are doubtful in terms of safety.

There has been known an air filter where an antibacterial compound makes use of an antibacterial effect which metal ion of silver, copper, zinc or the like possesses. Although such an air filter which makes use of metal ions may be excellent in terms of safety, the antibacterial effect is dissipated due to the oxidation of a surface of metal and hence, a stable antibacterial effect with time cannot be expected in such an air filter.

In general, a deodorizing means or method is roughly classified into: a method of neutralizing a substance such as ammonium or formaldehyde which causes odor by chemical reaction by spraying a deodorizing agent to the substance; and a method of absorbing odor by bringing such a substance into contact with a deodorizing agent. Since the air filter is used in a state where the air filter is arranged in the flow of air, the method of deodorizing by using an air filter belongs to the latter method.

A deodorizing agent used in the air filter is activated carbon, zeolite, calcium carbonate or the like in the form of a porous granular material. When monofilaments are formed by blending the porous granular material into a thermoplastic resin, it is found that only some fine pores exposed on front surfaces of the filaments have adsorption thus giving rise to a drawback that the air filter does not exhibit a deodorizing function efficiently and a drawback that the adsorption is largely lowered with the use of the air filter for a long period. Such prior art is disclosed in JP-A-11-309314.

### Citation List

### Patent Literature

PTL 1: JP-A-11-309314

### Summary of Invention

### Technical Problem

In the manufacture of the monofilament by spinning in general, in mixing a functional additive such as an antibacterial compound or a deodorizing agent in a raw material resin, usually, the functional additive is charged into an extruder directly or as a preset master batch such that the additive has the predetermined concentration. When a specification is adopted where a mixing amount of additive is small, it is difficult to uniformly disperse the additive in the raw material resin. On the other hand, when a specification is adopted where a mixing amount of additive is large, physical properties of the monofilaments is lowered and, particularly, stretch is liable to be lowered. Accordingly, some additional facilities become necessary in a net fabric forming step or durability of the completed air filter is insufficient. In this manner, the conventional air filter has drawbacks in terms of both the manufacture and the quality of the air filter.

Further, in case of the fibers formed by kneading a functional additive into a master batch in a raw material resin in advance and extruding such a raw material resin, it is often the case that the functional additive enters the inside of the fibers and does not appear on surfaces of the fibers so that the air filter cannot sufficiently exhibit functions that the additive has.

Further, when a functional additive is adhered to surfaces of fibers using an adhesive agent, it is not easy to surely fix the additive to the surfaces of the fibers, and the fibers are adhered to each other by the adhesive agent so that air passing holes may be clogged.

Still further, in the same manner as the fibers made of a material formed by kneading the adhesive into the resin, it is often the case that the additive agent enters the inside of the adhesive agent and is not exposed to a surface of the adhesive agent so that the air filter cannot sufficiently exhibit functions which the additive has.

Accordingly, it is an object of the present invention to provide a functional air filter which can overcome drawbacks on manufacture by spinning a fiber material with improved productivity and by acquiring excellent efficiency in net fabrication, and is hygienic, exhibits high safety and possesses deodorizing property as an acquired product by maintaining a sufficient function of suppressing breeding of mold and undesired bacteria for a long period.

### Means for Solving Task

(1) A functional air filter according to the present invention is a filter which is manufactured by, at intersections between wefts and warps made of a thermoplastic sheath-core type composite monofilament which is a composite fiber consisting of a core material and a sheath material made of a resin having a lower melting point than the core material, heat-fusing the sheath materials to each other, wherein the composite monofilament is configured such that some particles blended into the sheath material are exposed from a surface of the sheath material.
(2) The functional air filter according to the present invention, in the functional air filter having the above-mentioned constitution (1), is characterized in that, the particle is a mixed particle where fine particles are fixedly adhered to a surface of a coarse particle.
(3) The functional air filter according to the present invention is, in the functional air filter having the above-mentioned constitution (2), characterized in that the coarse particle is made of silica, alumina, zirconia, titania or a mixture of these elements, and the fine particle is a metal particle made of platinum, gold, silver, copper, nickel or stainless steel or a material which is produced by mixing catechin into the metal particle.
(4) The functional air filter according to the present invention is, in the functional air filter having any one of the above-mentioned constitutions (1) to (3), characterized in that, the exposure of the particles from the surface of the sheath material is provided by stretching the composite monofilament into which the particles are mixed in the longitudinal direction.
(5) The functional air filter according to the present invention is, in the functional air filter having any one of the above-mentioned constitutions (1) to (3), characterized in that, the exposure of the particles from the surface of the sheath material is provided by rotating the composite monofilament into which the particles are mixed.

### Advantage of Invention

The functional air filter according to the present invention adopts the composite monofilament as a mode of the fiber material and hence, the functional air filter can acquire required strength by the core material, and can acquire functions such as deodorizing property, antibacterial property, anti-oxidation property and the like by the sheath material.

Mixed particles to which fine particles are fixedly adhered are exposed on the surface of the coarse particle from the surface of the sheath material and hence, the functional air filter can directly exhibit excellent functions which the fine particles have such as excellent deodorizing property, excellent antibacterial property and anti-oxidation property.

Further, when the functional air filter is used in such a manner that the filter is washed with water, the mixed particles are not easily removed or separated from the sheath material and hence, the functions can be sustained for a long period.

Still further, the presence of the mixed particles also contributes to the enhancement of size stability and heat resistance of the composite monofilaments which constitute the air filter against a change in environment such as a change in temperature or humidity.

Still further, the mixed particles are blended into only the sheath material having a low melting point, and are exposed from the surface of the sheath material whose thickness is decreased by being pushed out by the core material having a high melting point and hence, the mixed particles are surely exposed on the surface of the air filter.

The sheath materials are heat-fused with each other by pressure-bonding an intersecting point between a weft and a warp and hence, a size of the air filter in the thickness direction becomes fixed and hence, the air filter can be easily cleaned automatically or manually.

### Brief Explanation of Drawings

Fig. 1 (a) to 1 (c) are cross-sectional views of a composite monofilament according to an embodiment of the present invention, wherein Fig. 1(a) is an explanatory view showing a state where mixed particles are embedded into a sheath material, Fig. 1 (b) is an explanatory view showing a state where the mixed particles are exposed on a surface of the sheath material by stretching the composite monofilament, and Fig. 1(c) is an explanatory view showing a state where the mixed particles are exposed on the surface of the sheath material by rotating the composite monofilament.
Fig. 2 is an explanatory view of a mixed particle showing a state where fine particles are fixedly adhered to a surface of a coarse particle.
Fig. 3 (a) and 3 (b) are cross-sectional explanatory views showing the structure of an air filter according to the embodiment of the present invention, wherein Fig. 3(a) shows a state where the air filter is formed by honeycomb weaving, and Fig. 3(b) is a schematic view showing a state where the air filter is formed by pressure-bonding wefts and warps.
Fig. 4 is an explanatory view showing another manufacturing method of the filter according to the embodiment of the present invention.
Fig. 5 is an explanatory view showing a method adopted by an evaluation test of the filter according to the embodiment of the present invention.
Fig. 6 is a graph showing deodorization evaluation against ammonium with respect to the filter according to the embodiment of the present invention.
Fig. 7 is a graph showing deodorization evaluation against acetaldehyde with respect to the filter according to the embodiment of the present invention.
Fig. 8 is a graph showing deodorization evaluation against tobacco with respect to the filter according to the embodiment of the present invention.
Fig. 9 is a graph showing evaluation with respect to cleaning of the filter according to the embodiment of the present invention.

### Mode for Carrying Out the Invention

As a sheath-core type thermoplastic resin which is a material of a composite monofilament used as a fiber material constituting the filter of the present invention, a polyolefin-based resin, a polyester-based resin, a polyamide-based resin, a polyacrylic resin, a polystyrene-based resin, a polyvinyl chloride-based resin and the like are named.

To be more specific, the sheath-core-type thermoplastic resin is a resin composition which is a single or a combination of polypropylene, high-density polyethylene, medium-density polyethylene, low-density polyethylene, straight-chain low-density polyethylene, ethylene-propylene copolymer, ethylene-vinyl acetate copolymer, ethylene-acrylic ester copolymer and the like.

### <Composite monofilament>

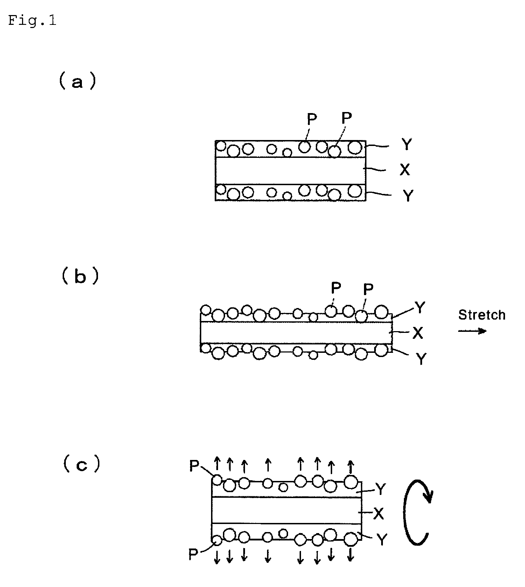

Fig. 1(a) to 1(c) are cross-sectional views of the composite monofilament according to the embodiment, wherein Fig. 1(a) is an explanatory view showing a state where mixed particles are embedded into a sheath material Y, Fig. 1(b) is an explanatory view showing a state where the mixed particles are exposed on a surface of the sheath material Y by stretching the composite monofilament, and Fig. 1(c) is an explanatory view showing a state where the mixed particles are exposed on the surface of the sheath material Y by rotating the composite monofilament.

As shown in Fig. 1 (b), the composite monofilament of this embodiment is a composite filament of a sheath-core joined type which is constituted of a core material X and the sheath material Y, and mixed particles P contained in the sheath material Y are exposed on a surface of the sheath material Y.

The composite monofilament of a sheath-core joined type may be a conventionally known sheath-core-type monofilament, and may be also any one of a concentric sheath-core-type composite monofilament, an eccentric sheath-core-type composite monofilament or a multi-core sheath-core-type composite monofilament.

### <Core material>

The core material X of the composite monofilament is formed using a thermoplastic resin having a relatively high melting point. Such a thermoplastic resin is a resin which is softened by heating the resin to a glass transition temperature or a melting point and can be formed into a desired shape.

### <Sheath material>

The sheath material Y of the composite monofilament is formed using a thermoplastic resin having a relatively low melting point, and mixed particles are blended into the sheath material Y. As an example of the thermoplastic resin having a low melting point which is used for forming the sheath material Y, a thermoplastic resin substantially equal to the thermoplastic resin having a high melting point which is used for forming the core material X can be used.

As the thermoplastic resin for forming the core material X and the sheath material Y, polyethylene, polypropylene, polyvinyl chloride, polyvinyliden chloride, polystyrene, polyvinyl acetate, Teflon (registered trademark), ABS resin, AS resin, acrylic resin and the like can be named. Any one of these thermoplastic resins can be used in the present invention.

Polyethylene is a polymer having the simplest structure where ethylene is polymerized, and high-density polyethylene, low-density polyethylene, ultra-low-density polyethylene, straight-chain low-density polyethylene or ultra-high molecular weight polyethylene can be named, and any one of these polyethylenes can be used in the present invention. Polyethylene may be not only homopolymer of ethylene but also propylene containing ethylene as a main component or a copolymer with α-olefin such as butene-1.

A melt index (MI) of polyethylene is set to 0.1 to 100. It is preferable to set the melt index of polyethylene to 0.2 to 80 in many cases. MI expresses a mass of a specimen which is extruded for 10 minutes under the condition where a temperature is 190°C and a load is 2160g and an orifice hole diameter is 2.092mm in terms of g.

Polypropylene used for forming the core material X and the sheath material Y is a polymer where propylene is polymerized, and polypropylene may be not only homopolymer of propylene but also ethylene which contains propylene as a main component or a copolymer with α-olefin such as butene-1. Amelt flow rate (MFR) of polypropylene is set to 0.3 to 400, and is more preferably set to 0.5 to 200.

A typical example of polypropylene is a propylene single polymer having a melting point of 150°C or above, for example. The composite filament where the core material X is formed using polypropylene having a high melting point is particularly preferable from a view point of spinning property, stretching property, physical properties (strength, size stability) and the like. A melt flow rate (MFR) expresses a mass of a specimen which is extruded for 10 minutes under the condition where a a temperature is 230°C and a load is 2160g and an orifice hole diameter is 2.092mm in terms of g.

In the composite monofilament having the above-mentioned constitution, as a thermoplastic resin used for forming the sheath material Y, a thermoplastic resin having a lower melting point than a thermoplastic resin for forming the core material X is used. For example, a resin having a melting point lower than a melting point of a thermoplastic resin for forming the core material X by 5°C or more, more preferably, 30°C or more, for example, can be used. When the core material X and the sheath material Y are formed using resins of the same kind respectively, the core material X having a high melting point can be formed by increasing an average molecular weight of the resin.

Further, in the sheath-core type composite monofilament, the melting point of the sheath material Y is lower than the melting point of the core material X and hence, mixed particles contained in the sheath material Y can be exposed from a surface of the sheath material Y by stretching the composite monofilament and by extruding the mixed particles using the hard core material X.

Further, since the melting point of the sheath material Y is low, intersections between wefts and warps of the sheath-core-type composite monofilament can be fused to each other when the monofilaments are formed into the filter. For example, it is usually preferable to fuse the sheath materials Y to each other at a temperature of approximately 160 to 240°C.

A kind of the resin for forming the core material and a kind of resin for forming the sheath material may be equal or may differ from each other.

Although a diameter of the sheath-core-type composite monofilament is not particularly limited and may be suitably decided, it is preferable to set the diameter of the sheath-core-type composite monofilament to approximately 50 to 400µm usually.

### <Mixed particles>

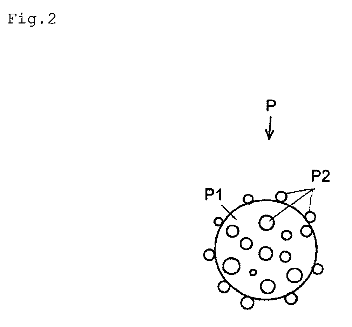

Although a kind of mixed particles P blended into the sheath material Y is not particularly limited, it is sufficient that the mixed particles P are not melted by heating at the time of stretching the composite monofilament. Accordingly, particles made of a resin, metal, glass, ceramic or the like are named as a kind of mixed particles P. By adding the mixed particles P into the sheath material Y, it is possible to impart a filter function to the filter. As the mixed particle, for example, as shown in Fig. 2, a particle which is formed by fixedly adhering fine particles P2 to a surface of the coarse particle P1 which constitutes a base can be used.

By fixedly adhering the fine particles having functions to the surface of the coarse particle, it is possible to prevent the fine particles having functions from being embedded into the resin so that the fine particles are exposed on the surface of the sheath material Y whereby the functions of the fine particles can be given to the filter.

### <Coarse particles>

A size of the coarse particle P1 is not particularly limited. However, a particle having an average particle size of approximately 1 to 100µm may be named as the coarse particle P1. A kind of the coarse particle is not particularly limited. However, it is sufficient that the particle is not melted at the time of molding a thermoplastic resin, and a particle made of ceramic, glass, resin, metal or the like may be named as the kind of the coarse particle. The shape of the coarse particle P1 is not particularly limited to a specific shape, and may be a spherical shape, an oval shape, a stereoscopic shape, a rectangular parallelepiped shape, a polygonal columnar shape, a flat shape or the like.

As a ceramic particle which constitutes the coarse particle, an alumina particle, a silica particle, a zirconia particle, a titania particle or the like is named. Further, various ceramics including mixtures of these components may be used for forming the coarse particle. Further, the ceramic particle may be also formed using a multivalent salt of an inorganic acid such as phosphorus, sulfuric acid, nitric acid, carbonic acid or the like, fluoride or silicofluoride of alkali metal or alkali earth metal, colloidal silica or organosilicasol which uses organic solvent such as alcohol as a medium.

Further, various clay minerals, oxides, hydrides, composite oxides, nitrides, carbides, silicides, bolides, zeolites, cristobalites, diatom earths, multivalent metal salts of silicates and the like can be also used.

As clay minerals, kaoline, agalmatolite, sericite, bentonite and the like are named.

As oxides, alumina, titania, silica, zirconia, magnesia and the like are named.

As hydrides, hydride of aluminum, hydride of zinc, hydride of magnesium, hydride of calcium, hydride of manganese and the like are named.

As composite oxides, aluminum potassium sulfate, mica and the like are named. As nitrides, silicon nitride, boron nitride and the like are named. As carbides, silicon carbide, boron carbide and the like are named.

As multivalent metal salts of silicates, aluminum salt, magnesium salt, calcium salt, manganese salt and the like are named.

### <Hollow body>

Further, as the coarse particle, it is also possible to use a coarse particle in the form of a hollow body. The hollow body is a body in which one, two or more independent air bubbles which are not communicated with the outside (closed hollow portion) are formed in the body. For example, ceramic balloon formed using silica, alumina, titania, zirconia, calcium carbonate or the like as a raw material, glass balloon formed by using glass as a raw material, a shirasu (volcanic ash) balloon or the like may be named. Further, a pearlite foamed body, fly ash balloon can be also used. A size (inner diameter) of a hollow portion of the hollow body is not particularly defined. With the use of the hollow body, the weight of the composite filament can be reduced.

Further, with the use of the coarse particle having closed hollow portions, when a sheath material of a composite monofilament formed using a thermoplastic resin having a low melting point is melted, the coarse particle easily floats on a surface of the sheath material and hence, the coarse particle is easily exposed on the surface of the sheath material as a mixed particle.

Further, since the hollow body is a closed-type hollow body and hence, a mixed particle has no water absorbency whereby it is possible to prevent the filter from absorbing moisture.

### <Fine particles P2>

As fine particles P2 carried on a surface of a coarse particle, particles having a smaller particle size than the above-mentioned coarse particle, for example, particles having an average particle size of approximately 1 to 10nm can be named.

Further, although a kind of fine particles is not particularly limited, metal particles made of platinum, gold, silver, copper, nickel, stainless steel or the like can be named. Approximately 10 to 30 mass% of catechin may be mixed into these metal particles.

By setting a particle size of fine particles at nano order, it is possible to impart a function which fine particles have to mixed particles. For example, while it is thought that metal particles made of, for example, platinum, gold, silver or the like have a catalytic function and an antibacterial function, by fixedly adhering fine particles on a surface of a coarse particle, it is possible to efficiently make metal particles made of expensive platinum, gold, silver or the like exposed on a surface of a sheath material and hence, it is possible to impart a catalytic effect such as an antibacterial function, a deodorizing function or anti-oxidation function to the filter for a long period.

### <Ratio between coarse particle and fine particles>

With respect to the relationship between a coarse particle and fine particles in a mixed particle, it is desirable to set an amount of fine particles to 0.1 to 10 parts by mass for 100 parts by mass of the coarse particle. An amount of fine particles is preferably set to 0.1 to 5 parts by mass, and it is more preferable to set an amount of fine particles to 0.2 to 1 parts by mass. When the ratio of fine particles is excessively small, the filter cannot sufficiently exhibit desired functions such as antibacterial function, a deodorizing function, an anti-oxidation function, while when the ratio of fine particles is excessively large, a balance between the fine particles and the coarse particle collapses, and also a manufacturing cost is pushed up.

To make a coarse particle carry fine particles on a surface thereof, for example, the mixture of fine particles and coarse particles is sintered by heating thus forming mixed particles where fine particles are strongly and fixedly adhered to a surface of the coarse particle and hence, even when fine particles are exposed on a surface of a sheath material without being embedded in a resin, it is possible to prevent the removal or falling of the fine particles.

Further, the following method is also applicable to fixedly adhere the fine particles to the surfaces of the coarse particles. That is, fine particles made of platinum or the like are brought into a colloidal state using a colloid forming agent (dispersing liquid containing fine particles), and the coarse particles, a binding agent (for example, colloidal silica) and dispersive medium (water, alcohol or the like) are mixed into the dispersing liquid.

As the above-mentioned colloid forming agent, a thickening agent, a surfactant, a carboxyl group-containing compound which contains a carboxyl group in the chemical structure can be named. A polyacrylic acid (including salt such as Na, K), a polymethacrylic acid (including salt such as Na, K), polyacrylic acid ester, polymethacrylic acid ester, polyvinylpyrrolidone, (particularly, poly-1-vinyl-2-pyrrolidone), polyvinyl alcohol, amino pectin, pectin, methyl cellulose, methyl sulose, glutathione, cyclodextrin, polycyclodextrin, dodecanthiol, an organic acid (a hydroxy carboxylic acid such as a citric acid), glycerine fatty acid ester (polysorbate), cationic micellar-cetyl trimethyl ammonium bromide, a surfactant (anionic, cationic, amphoteric, nonionic), alkali metal salt of alkylsulfuric acid ester and compounds thereof may be exemplified.

When the colloid forming agent is a carboxyl group-containing compound, it is desirable to make fine particles contain a carboxyl group such that the number of molecules of the carboxyl group becomes approximately 80 to 180 with respect to the number of molecules of platinum. With respect to the content of colloidal silica as a binder, it is desirable that a mass of solid amount is 10mass% or more and 50mass% or below with reference to the whole colloid forming agent, and it is more preferable to set the mass of the solid amount to 10mass% or more and 30mass% or below. Colloidal silica means silica particles having a particle size of approximately 1nm to 1µm.

In the above-mentioned fine particle containing dispersion liquid, when fine particles are made of platinum, for example, a solution which is produced by dissolving platinum metal salt and a protective agent (for example, organic acid) into a mixed liquid of water and alcohol is refluxed so as to precipitate platinum fine particles thus preparing fine particle containing dispersion liquid. Thereafter, the dispersion liquid may be replaced with alcohol (ethanol or the like).

As a method of replacing a dispersion liquid with alcohol, a method where an operation of evaporating a part of dispersion medium before replacement and, thereafter, adding a dispersion medium (alcohol or the like) after replacement is repeated can be exemplified.

A fine particle containing dispersion liquid, coarse particles and a binder are mixed to each other thus forming a liquid substance in a slurry state, fine particles in a colloidal shape (fine particle containing dispersion liquid) is fixedly adhered to a surface of the coarse particle (a product produced by adhesion being referred to as fixedly adhered substance), the fixedly adhered substance is pulverized and is dried, and a dispersion medium in the fine particle containing dispersion liquid in the fixedly adhered substance is removed whereby fine particles are fixedly adhered to (carried on) the surface of the coarse particle.

After fixedly adhering the fine particle containing dispersion liquid to the surface of the coarse particle, the dispersion medium is removed from the surface of the coarse particle (oxidation removing step). In the removal of the dispersion medium, a colloid forming agent is removed by oxidation by heating under an oxidization atmosphere. Here, colloidal silica which functions as a binder is melted or softened so that fine particles are carried on the surface of the coarse particle.

It is desirable to set a heating temperature in such a step, by taking into account a melting or softening temperature of the binder, to approximately 800°C to 1100°C. It is more desirable to set the heating temperature to 900°C to 1000°C.

Heating time can be set to an appropriate value corresponding to time necessary for removing a colloid forming agent by oxidation. For example, the heating time may be set to approximately 1 hour to 3 hours.

As a method of pulverizing the above-mentioned fixedly adhered substance, spray drying treatment (spray drying method) may be adopted. The spray drying treatment is a treatment method where a liquid substance in a slurry state which is a raw material is formed into a fine powder state, and is a method of acquiring dried powdery material by spraying a liquid substance in a slurry state into hot blast and, at the same time, drying the liquid substance by heating.

In this embodiment, as a condition of spraying and drying by heating, a heating temperature may be set to a temperature at which a dispersion medium can be speedily removed by evaporation, for example, approximately 180°C to 250°C.

### <Ratio between thermoplastic resin and mixed particles>

In the composition of such raw materials, it is important that 0.2 to 5.0% by weight of mixed particles is blended into a thermoplastic resin. That is, when an amount of mixed particles is smaller than 0.2% by weight, the air filter cannot acquire a sufficient antibacterial function and a sufficient deodorizing function. To the contrary, even when an amount of mixed particles is increased by exceeding 5.0% by weight, there arise drawbacks which decrease productivity such as a drawback where while the above-mentioned functions are no more improved, so that a material cost is pushed up in a wasteful manner and a drawback that it is difficult to acquire the uniform dispersion of mixed particles in the resin.

With respect to a ratio between a thermoplastic resin and mixed particles in the sheath material, it is desirable to set an amount of mixed particles to 1 to 50 parts by mass for 100 parts by mass of thermoplastic resin. It is preferable to set an amount of mixed particles to 2 to 30 parts by mass. When a blending amount of mixed particles is excessively small, the filter cannot sufficiently exhibit desired functions such as a deodorizing function, antibacterial function, a bio active function and an anti-oxidation function. On the other hand, even when a blending amount of the mixed particles is excessively large, not only that functions are not improved exceeding a fixed level but also disadvantages such as lowering of productivity of composite monofilaments which constitute the filter or lowering of strength and texture become conspicuous.

### <Ratio between core material and sheath material>

A ratio between the core material X and the sheath material Y in the composite monofilament is set, as expressed by a mass ratio, such that the core material X : the sheath material Y = 30 : 70 to 80 : 20, preferably, 35 : 65 to 75 : 25. This is because when the ratio of the sheath material Y is small, the ratio of the mixed particles becomes small and hence, desired functions cannot be sufficiently acquired.

On the other hand, when the ratio of the sheath material Y is large, the mixed particles are embedded into the sheath material Y after stretching thus increasing a possibility that the mixed particles are not exposed on the surface of the sheath material Y.

### <Method of manufacturing composite monofilament>

The composite monofilament according to this embodiment can be manufactured by performing co-extrusion molding of a thermoplastic resin having a high melting point and a thermoplastic resin having a low melting point in which mixed particles are blended such that the thermoplastic resin having a high melting point forms the core material X, and the thermoplastic resin having a low melting point into which mixed particles are blended forms the sheath material Y.

The spinning of a composite monofilament is performed in such a manner that, using extruders in two series and a filament forming device provided with a composite nozzle of the sheath-core structure having approximately concentric ejection holes, a core layer content resin and a sheath layer content resin are respectively charged into the extruders, the resins are extruded from the extruders in a molten state and are cooled and, thereafter, the resins are heated and stretched through a hot-blast stove, heat rolls, a water bath or the like, and the resin is subjected to slackening treatment.

When necessary, an assistant such as an oxidation inhibitor, an ultraviolet ray absorbing agent, a coloring agent, a lubricant, an antistatic agent, a delustering agent, a fluidity improving agent, a plasticizer or an incombutible material may be added to a thermoplastic resin of the sheath material or the core material. Particularly, with respect to the thermoplastic resin of the sheath material into which mixed particles are blended, it is preferable to assure the uniform distribution of the mixed particles by blending a forming assistant which is effective for enhancing the aggregation prevention property or the dispersibility including a metal soap together with a stabilizer such as an oxidation inhibitor in combination.

Further, a proper amount of metal ion source such as copper salt, iron salt, calcium salt, titanium salt, aluminum salt, silver salt, tin salt, zinc salt, chromium salt or cobalt salt may be allowed to coexist with the mixed particles so as to enhance carrying property of the mixed particles.

As shown in Fig. 1(b), the manufactured unstretched composite monofilament is stretched by the subsequent stretching treatment thus decreasing a wall thickness of the sheath material Y whereby mixed particles blended in the sheath material Y are exposed on a surface of the sheath material Y. At this point of time, a melting point of the thermoplastic resin which forms the sheath material is lower than a melting point of the thermoplastic resin which forms the core material. Accordingly, the sheath material is further stretched so that the wall thickness of the sheath material is decreased and hence, some mixed particles blended into the sheath material are exposed on the surface of the sheath material.

Although the wall thickness of the sheath material after stretching is not particularly limited, it is desirable to set the wall thickness of the sheath material smaller than an average particle size of the mixed particle.

Although a stretching magnification is not particularly limited, when the magnification is excessively small, a ratio that mixing particles blended in the sheath material is exposed on the surface of the sheath material becomes insufficient and hence, it is desirable to set the stretching magnification to 5 times or more.

On the other hand, when the stretching magnification is excessively large, troubles including a trouble that interlayer peeling is liable to be generated in a bonding interface between the core and the sheath occur and hence, it is desirable to set an upper limit of the stretching magnification to approximately 10 times in general.

Further, it is desirable to set a stretching temperature to a softening temperature of the thermoplastic resin which forms the sheath material or more.

By blending titanium oxide or metal particles which are effective as a photocatalyst into the composite monofilament, and by exposing titanium oxide or metal particles on the surface of the sheath material Y, it is possible to acquire a raw material having an extremely efficient photocatalytic function.

### <Rotation>

As a means for exposing particles embedded in the sheath material from the surface of the sheath material, besides the above-mentioned stretching, as shown in Fig. 1(c), the particles can be exposed from the surface of the sheath material by making use of a centrifugal force generated by rotating the sheath-core-type composite monofilament while grasping one end of the monofilament after extrusion molding. In this case, with respect to the exposure condition, the higher a rotational speed, the more the particles are exposed. However, the exposure condition is suitably determined also by taking into account the relationship with strength of the composite monofilament. Usually, it is desirable to perform the rotation of the composite monofilament in an atmosphere close to a melting temperature of the sheath material at a rotational speed of 100 to 500rpm for 1 to 2 seconds.

### <Filter>

With respect to the filter according to the embodiment of the present invention, a functional filter (a filter of an air conditioner, an air cleaner, a vacuum cleaner or the like) can be manufactured using the above-mentioned composite monofilament.

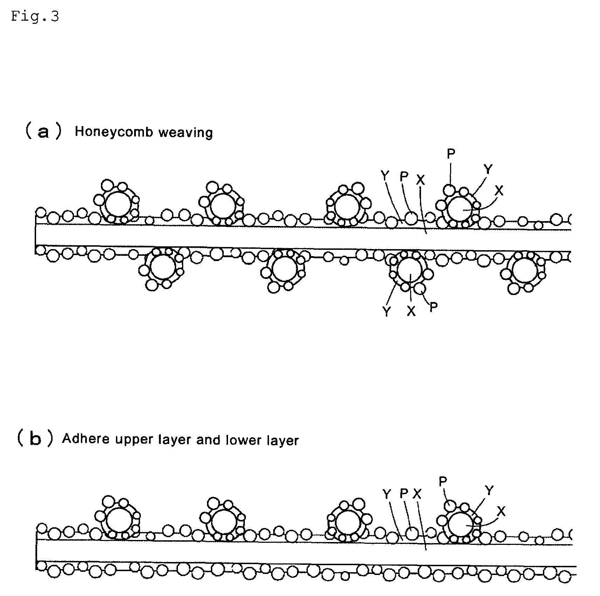

Fig. 3(a) and Fig. 3(b) are cross-sectional explanatory views showing the structure of the filter according to this embodiment, wherein Fig. 3(a) shows a state where the filter is formed by honeycomb weaving, and Fig. 3(b) is a schematic view showing a state where the filter is formed by pressure bonding wefts and warps.

### <Net fabric structure>

A fiber material which represents the composite monofilament obtained in this manner constitutes a net fabric material and forms an air filter. With respect to the net fabric structure, the structure used in general, to be more specific, with respect to a woven fabric, plain weaving, leno weaving, mock leno weaving, gauze and leno weaving and the like are named.

Further, from a viewpoint of size stability in handling in addition to elasticity, flexibility, ventilating ability and dust collecting property which an air filter is required to possess, it is desirable to use a honeycomb woven structural body formed of a fiber material having fineness of 80 to 500dr particularly (see fiber terms (fabric section) in JIS-L0206-1976) (see Fig. 3(a)).

The honeycomb woven structural body is woven in series using a Sulzer type loom or the like. The honeycomb woven structural body is characterized by having the stereoscopic structure where concave and convex portions are formed on front and back surfaces of the structure. In the air filter according to the present invention, weaving density of warps and wefts can be set to 30 to 75 lines per inch.

### <Thermal bonding>

In the present invention, intersections where wefts and warps of the sheath materials intersect with each other are adhered by thermal bonding after the filter is formed. This adhesion by thermal bonding is performed such that threads are woven into a filter shape, and the obtained woven fabric is heated simultaneously with weaving or after weaving at a temperature at which the sheath material having a low melting point in a thread form is melted or softened and at the temperature at which the core material having a high melting point is not softened. Due to such adhesion by thermal bonding, a thickness of the filter becomes a fixed value so that the maintenance such as cleaning of the filter is facilitated.

As a device for performing thermal bonding of the intersections of the threads of the woven fabric by heating, a hot-blast-type heater, an infrared ray heater, a far infrared heater, a high pressure vapor heater, a ultrasonic heater, a heated-roll type heater, a thermal pressure bonding roll type heater and the like can be named. Further, the combination of a plurality of these heaters can be also used.

The above-mentioned thermal bonding of the intersections of the wefts and warps of the sheath materials may be also performed as follows. That is, instead of the fabric shown in Fig. 3(a), the structure where wefts are arranged parallel to each other and the structure where warps are arranged parallel to each other are prepared as an upper layer and a lower layer respectively, the upper and lower layer are made to overlap with each other vertically thus forming a filter in a net shape and, thereafter, the intersections of these wefts and warps are thermally bonded to each other.

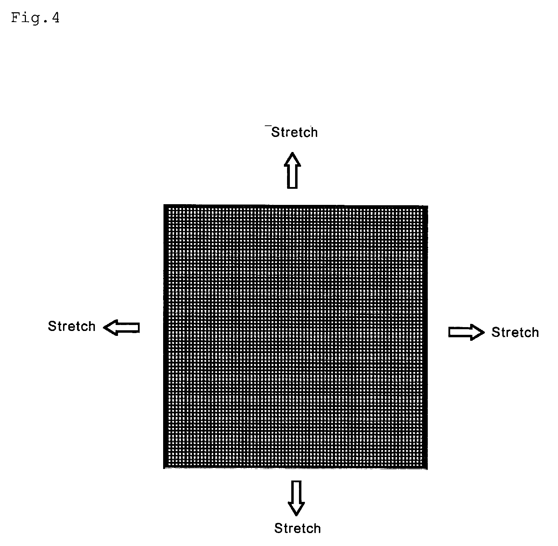

It may be also possible to adopt a method where the filter is stretched after the filter in a net shape is formed. As shown in Fig. 4, it may be possible that the filter is formed by weaving wefts and warps formed of a composite monofilament and, thereafter, these wefts and warps are stretched vertically and laterally thus exposing mixed particles embedded in the sheath material from the surface of the sheath material.

### Examples

Next, the present invention is explained in further detail in conjunction with examples.

### <Example 1>

Dispersing liquid containing fine particles of platinum is fixedly adhered to surfaces of the coarse particles P1 using a spray dryer. That is, particles formed of silica having an average particle size of 1µm and a platinum nano colloid dispersing liquid having a volume average particle size of approximately 5nm (= dispersing liquid containing fine particles, made by Apt Co. Ltd., content of platinum: 20µg/0.1g, a volume average particle diameter of a platinum fine particle being 5µm, and colloid forming agent being a citric acid) are mixed to each other such that a mass ratio of particles to platinum nano colloid dispersing liquid becomes 3 to 7.

Colloidal silica which is composed of 35.5% of silica (SiO₂ and 64.5% of H₂O is added to the mixed liquid as a binder by the same amount as the mixed liquid in terms of parts by mass. The mixed liquid is sprayed into the inside of a tank and is dried with a hot blast at 200°C using a spray drier. The obtained powder is collected and, thereafter, is put into a ceramic type container and is heated at a temperature of approximately 900 to 1000°C in an electric furnace for an hour (removal of a colloid forming agent by oxidation).

As a result, a citric acid which constitutes a colloid forming agent is oxidized and volatilized whereby mixed particles P are formed where platinum nano fine particles (fine particles P2) having a volume average particle size of 5nm are fixedly adhered to a surface of silica having a particle size of approximately µm (coarse particle P1).

Next, as a thermoplastic resin for forming the core material X, polypropylene (PP) having a melting point of 163°C and an MFR of 3.1 is prepared. Polypropylene (PP) having a melting point of 128°C and an MFR of 17.3 is prepared as a thermoplastic resin for forming a sheath material. Then, the above-mentioned mixed particles P are mixed into the thermoplastic resin for forming the sheath material (5 parts by mass of mixed particles P being blended to 100 parts by mass of sheath material resin). Using two set of extruders having a composite die, the sheath material Y is formed at a temperature of 205°C and the core material X is formed at a temperature of 230°C by co-extrusion molding by a filament forming device provided with a composite nozzle having the sheath-core structure with approximately concentric discharge holes. A mass ratio between the core material X and the sheath material is set such that core : sheath = 2 : 1.

Next, the formed material by extrusion is stretched at a stretching temperature of 230°C and at a stretching magnification of approximately 6 times thus forming a composite monofilament of 300 denier where the mixed particles P are exposed from the surface of the sheath material. At this point of time, a size of the core material X is 200 denier.

Using the composite monofilaments as wefts and warps, a honeycomb woven structural body material where one side of each honeycomb structural unit is 5.2mm and a thickness of each honeycomb structural unit is 2.2mm is woven at beating density of 60×60 per inch, and the honeycomb woven structural body material is used as the air filter of this example.

### <Evaluation of filter>

An effect of deodorizing ammonium, acetaldehyde and tobacco is investigated with respect to the filter of the example. A measuring method of a deodorizing test is carried out in accordance with "deodorizing performance test" in JEM-1467-1995 which is the standard with respect to "household air cleaner" determined by the Japan Electrical Manufacturer's Association.

### <Filter>

The following three kinds of filters are prepared.
(a) Filter according to the example of the present invention
(b) Conventional product (filter formed of polypropylene composite monofilament fibers, no kneading of mixed particles into fibers)
(c) Empty operation (no filter)

The above-mentioned filters are formed by weaving 34 pieces of wefts and 33 pieces of warps by honeycomb weaving.

### <Test method (deodorizing test method and content of evaluation)>

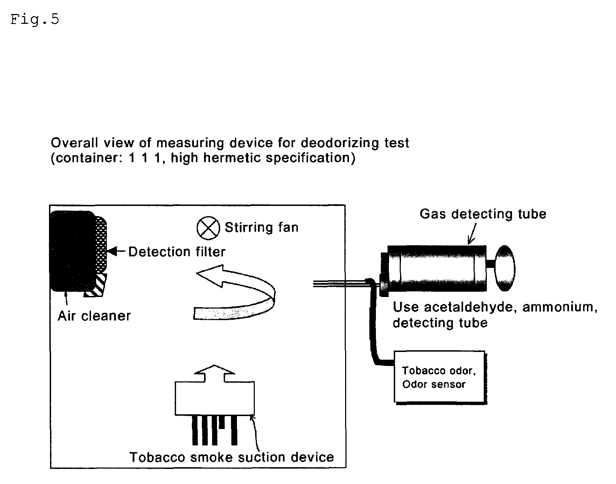

As shown in Fig. 5, a hermetically sealable container having an approximately cubic shape and a capacity of 1mm³ is prepared, an air cleaner which can be remote-controlled from outdoors is mounted in the container, and two sheets of deodorizing filters which constitute a specimen having a size of 300mm X 300mm are mounted on a mounting portion of a full-face filter for every measurement, and a circulation system is prepared where the distribution of odor in the container becomes uniform.

As a preparation for measurement of odor, firstly, five pieces of tobaccos are mounted on a smoke suction device, all five pieces of tobaccos are fired simultaneously and are burned for approximately 6 to 8 minutes. However, at the point of time that the first one tobaccos reaches the filter, the smoke suction device is stopped thus making remaining tobaccos naturally generate smokes. The smoke suction device is arranged at the center of a floor in a test space.

During a period where smoke from tobacco is sucked and during a period where smoke is generated from tobacco, the operation of the air cleaner on which the filter is mounted is stopped, and the operation of the air cleaner is started at a point of time that the last tobacco is burned out by a remote control.

With respect to the measurement of odors, after approximately five minutes elapse from starting the operation of the air cleaner, as initial density, odor from tobacco is measured by an odor sensor, or acetaldehyde and ammonium are measured using a gas detection tube.

The measurement of the deodorizing performance is performed such that the air cleaner is stopped after being operated for 30 minutes and for 60 minutes respectively, and deodorizing performance is measured thereafter.

With respect to acetaldehyde, a deodorizing test is performed separately and individually from the tobacco smoke generation test using an acetaldehyde standard liquid.

### <Deodorizing performance result>

### (1) Ammonium

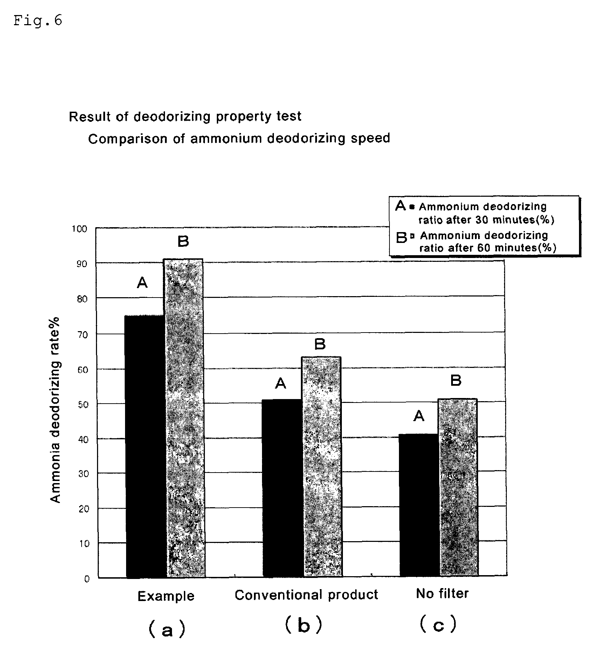

As shown in Fig. 6, the filter of the example exhibits a deodorizing effect approximately 1.5 times as high as a deodorizing effect of the conventional filter. In the drawing, A and B indicate deodorizing rates η of respective odor components after the lapse of 30 minutes and after the lapse of 60 minutes respectively which are obtained by the following formulae.

Deodorizing rate (%) (axis of ordinates)
A : η30 = (1-C30/C0)×100
B : η60 = (1-C60/C0)×100

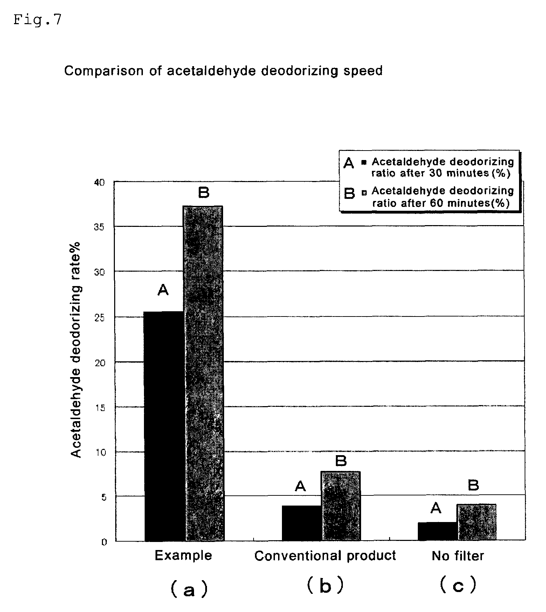

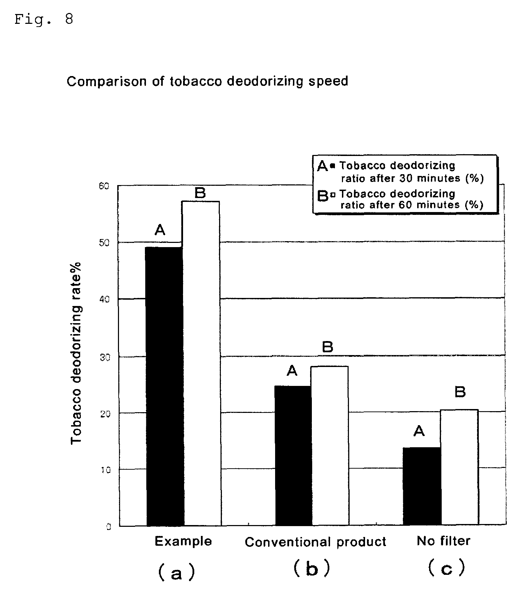

The same goes for Fig. 7 and Fig. 8.

### (2) Acetaldehyde

As shown in Fig. 7, the filter of the example exhibits a deodorizing effect approximately 3 to 5 times as high as a deodorizing effect of the conventional filter.

### (3) Tobacco

As shown in Fig. 8, the filter of the example exhibits a deodorizing effect approximately 2 times as high as a deodorizing effect of the conventional filter.

### <Influence on deodorizing performance by cleaning>

The influence on deodorizing performance by cleaning is investigated. The following cycle test is repeated. In an ammonium deodorizing test measurement cycle for 30 minutes, a filter is cleaned at a point of time 1 cycle is finished and, after natural drying, a test is carried out with a new cycle again.

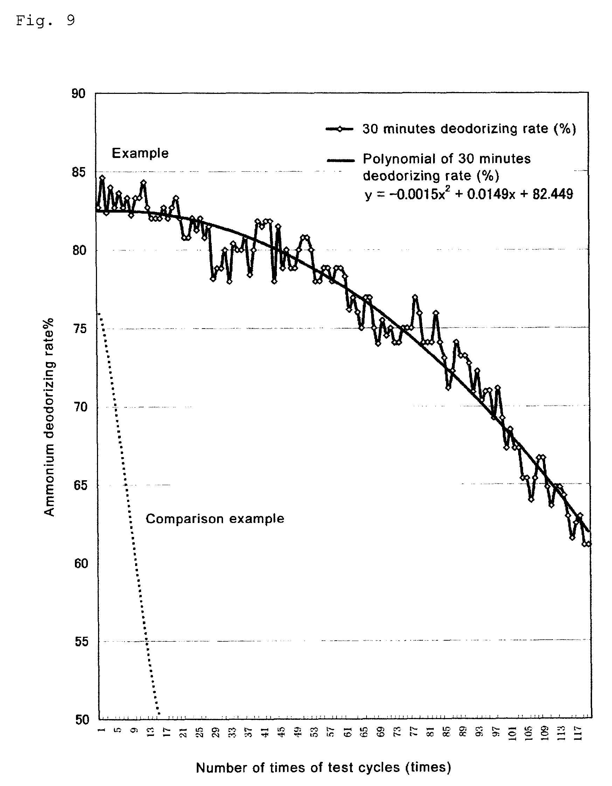

As a result, as shown in Fig. 9, even when the filter is washed with water 100 times, a deodorizing rate is maintained at 70%, and there is no remarkable lowering from the initial 83%. From this result, it is understood that there is no peeling of mixed particles exposed from the sheath material.

On the other hand, in a comparison example, a honeycomb woven filter where catechin is kneaded into usual fibers is used. A deodorizing effect is lowered below 50% at 15 cycles, and so that a deodorizing effect is dissipated.

### <Example 2>

Shirasu balloons formed of a hollow body having an average particle size of approximately 2µm are used as coarse particles P1 in the example 2. The filter is formed in the same manner as the example 1 except for that a composite monofilament is formed by exposing mixed particles P on a surface of a sheath material by rotating the composite monofilament as shown in Fig. 1(c) (rotational speed: 100 times/minute). When the deodorizing effect is evaluated in the same manner as the example 1, the example 2 can acquire the substantially same advantageous effects as the example 1.

### <Example 3>

A filter is formed in the same manner as the example 1 except for a point that shirasu balloons formed of a hollow body having an average particle size of approximately 2µm are used as coarse particles P1 in the example 3 and 10 mass% of catechin powder is added to a platinum nano colloid dispersion liquid as fine particles P2.

When the deodorizing effect is evaluated in the same manner as the example 1, the example 2 can acquire the substantially same advantageous effects as the example 1.

### Industrial Applicability

The functional filter of the present invention can acquire required strength by the core material, and can acquire functions such as deodorizing property, antibacterial property and anti-oxidation property by the sheath material. Further, the functional filter of the present invention uses composite monofilaments where particles are exposed from a surface of the sheath material and hence, the functional filter can directly exhibit functional effects which the particles have whereby the industrial applicability of the functional filter is extremely high.

### Explanation of symbols

- P:: mixed particle
- P1:: fine particle
- P2:: coarse particle
- X:: core material
- Y:: sheath material

## Claims

1. A functional air filter which is manufactured by, at intersections between wefts and warps made of a thermoplastic sheath-core type composite monofilament which is a composite fiber consisting of a core material and a sheath material made of a resin having a lower melting point than the core material, heat-fusing the sheath materials to each other, wherein
the composite monofilament is configured such that some particles blended into the sheath material are exposed from a surface of the sheath material.

2. The functional air filter according to claim 1, wherein the particle is a mixed particle where fine particles are fixedly adhered to a surface of a coarse particle.

3. The functional air filter according to claim 2, wherein the coarse particle is made of silica, alumina, zirconia, titania or a mixture of the elements, and the fine particle is a metal particle made of platinum, gold, silver, copper, nickel or stainless steel or a material which is produced by mixing catechin into the metal particle.

4. The functional air filter according to any one of claims 1 to 3, wherein the exposure of the particles from the surface of the sheath material is provided by stretching the composite monofilament into which the particles are mixed in the longitudinal direction.

5. The functional air filter according to any one of claims 1 to 3, wherein the exposure of the particles from the surface of the sheath material is provided by rotating the composite monofilament into which the particles are mixed.
